(19) Europäisches Patentamt
European Patent Office
Office européen des brevets

(11) EP 3 717 448 B1

(12) # EUROPÄISCHE PATENTSCHRIFT

(45) Veröffentlichungstag und Bekanntmachung des Hinweises auf die Patenterteilung:
**06.10.2021 Patentblatt 2021/40**

(21) Anmeldenummer: **18800675.3**

(22) Anmeldetag: **19.11.2018**

(51) Int Cl.:
***C07C 209/02*** (2006.01) ***C07C 209/16*** (2006.01)
***C07C 211/11*** (2006.01) ***C07C 211/14*** (2006.01)
***B01J 23/00*** (2006.01)

(86) Internationale Anmeldenummer:
**PCT/EP2018/081728**

(87) Internationale Veröffentlichungsnummer:
**WO 2019/105782 (06.06.2019 Gazette 2019/23)**

(54) **VERFAHREN ZUR KONTINUIERLICHEN HERSTELLUNG VON 1,2-PROPYLENDIAMIN (1,2-PDA) UND DIMETHYLDIETHYLENTRIAMIN (DMDETA)**

METHOD FOR THE CONTINUOUS PRODUCTION OF 1,2-PROPYLENDIAMINE (1,2-PDA) AND DIMETHYLDIETHYLENTRIAMINE (DMDETA)

PROCÉDÉ DE PRÉPARATION CONTINUE DE 1,2-PROPYLÈNE DIAMINE (1,2-PDA) ET DE DIMÉTHYL DIÉTHYLÈNETRIAMINE (DMDETA)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **30.11.2017 EP 17204658**

(43) Veröffentlichungstag der Anmeldung:
**07.10.2020 Patentblatt 2020/41**

(73) Patentinhaber: **BASF SE**
**67056 Ludwigshafen am Rhein (DE)**

(72) Erfinder:
- **EIDAMSHAUS, Christian**
  **67056 Ludwigshafen (DE)**
- **MELDER, Johann-Peter**
  **67056 Ludwigshafen (DE)**
- **PASTRE, Joerg**
  **67056 Ludwigshafen (DE)**
- **PALLASCH, Hans-Juergen**
  **67056 Ludwigshafen (DE)**

(74) Vertreter: **BASF IP Association**
**BASF SE**
**GBI-C006**
**67056 Ludwigshafen (DE)**

(56) Entgegenhaltungen:
- **DATABASE WPI Week 201565 Thomson Scientific, London, GB; AN 2015-485522 XP002781352, -& CN 104 693 037 A (CAS DALIAN CHEM & PHYSICAL INST) 10. Juni 2015 (2015-06-10) in der Anmeldung erwähnt**
- **DATABASE WPI Week 201449 Thomson Scientific, London, GB; AN 2014-N90188 XP002781353, & CN 103 819 344 A (XI'AN MODERN CHEM RES INST) 28. Mai 2014 (2014-05-28)**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Anmeldung betrifft ein Verfahren zur kontinuierlichen Herstellung von 1,2-Propylendiamin (1,2-PDA) und Dimethyldiethylentriamin (DMDETA) durch Umsetzung von Monoisopropanolamin (MIPOA) mit Ammoniak in Gegenwart von Wasserstoff und eines geträgerten Heterogen-Hydrierkatalysators (Katalysator), wie in Anspruch 1 definiert.

STAND DER TECHNIK

1,2-PDA findet unter anderem Verwendung als Zwischenprodukt bei der Herstellung von Kraftstoffadditiven, Tensiden, Arznei- und Pflanzenschutzmitteln, Härtern für Epoxyharze, Katalysatoren für Polyurethane, Zwischenprodukte zur Herstellung quaternärer Ammoniumverbindungen, Weichmachern, Korrosionsinhibitoren, Kunstharzen, Ionenaustauschern, Textilhilfsmitteln, Farbstoffen, Vulkanisationsbeschleunigern und/oder Emulgatoren. DMDETA kann beispielsweise als Epoxidhärter eingesetzt werden.

WO 03/051508 A1 (Huntsman Petrochemical Corp.) betrifft Verfahren zur Aminierung von Alkoholen unter Verwendung von spezifischen Cu/Ni/Zr/Sn - haltigen Katalysatoren, die in einer weiteren Ausgestaltung Cr statt Zr enthalten (siehe Seite 4, Zeilen 10-16). Die in dieser WO-Anmeldung beschriebenen Katalysatoren enthalten kein Aluminiumoxid und kein Kobalt.

WO 2008/006750 A1 (BASF AG) betrifft bestimmte Pb, Bi, Sn, Sb und/oder In - dotierte, zirkoniumdioxid-, kupfer-, nickel- und kobalthaltige Katalysatoren und ihre Verwendung in Verfahren zur Herstellung eines Amins durch Umsetzung eines primären oder sekundären Alkohols, Aldehyds und/oder Ketons mit Wasserstoff und Ammoniak, einem primären oder sekundären Amin. Aluminiumoxid-Träger werden nicht gelehrt.

WO 2009/080507 A1 (BASF SE) betrifft bestimmte Sn- und Co-dotierte, zirkoniumdioxid-, kupfer- und nickelhaltige Katalysatoren und ihre Verwendung in Verfahren zur Herstellung eines Amins durch Umsetzung eines primären oder sekundären Alkohols, Aldehyds und/oder Ketons mit Wasserstoff und Ammoniak, einem primären oder sekundären Amin. Aluminiumoxid-Träger werden nicht gelehrt.

WO 2009/080506 A1 (BASF SE) beschreibt bestimmte Pb, Bi, Sn, Mo, Sb und/oder P - dotierte, zirkoniumdioxid-, nickel- und eisenhaltige Katalysatoren und ihre Verwendung in Verfahren zur Herstellung eines Amins durch Umsetzung eines primären oder sekundären Alkohols, Aldehyds und/oder Ketons mit Wasserstoff und Ammoniak, einem primären oder sekundären Amin. Aluminiumoxid-Träger werden nicht gelehrt. Bevorzugt enthalten die Katalysatoren kein Cu und kein Co.

WO 2009/080508 A1 (BASF SE) lehrt bestimmte Pb, Bi, Sn und/oder Sb - dotierte, zirkoniumdioxid-, kupfer-, nickel-, kobalt und eisenhaltige Katalysatoren und ihre Verwendung in Verfahren zur Herstellung eines Amins durch Umsetzung eines primären oder sekundären Alkohols, Aldehyds und/oder Ketons mit Wasserstoff und Ammoniak, einem primären oder sekundären Amin. Aluminiumoxid-Träger werden nicht gelehrt.

WO 2009/114438 A2 (Huntsman Petrochem. Corp.) betrifft die Aminierung von Cyclohexandimethanol in Gegenwart von Wasserstoff und $ZrO_2$-geträgerten Metallkatalysatoren, z. B. $ZrO_2$/Cu/Ni/Sn.

1,2-PDA lässt sich durch Umsetzung von Monoisopropanolamin (MIPOA) mit Ammoniak und Wasserstoff in Gegenwart in Gegenwart eines geeigneten Katalysators herstellen.

WO 2011/067199 A1 (BASF SE) beschreibt ein Verfahren zur Herstellung eines Amins durch Umsetzung eines primären oder sekundären Alkohols, Aldehyds oder Ketons mit Wasserstoff und Ammoniak in Gegenwart eines geträgerten kupfer-, nickel- und kobalthaltigen Katalysators, der im Bereich von 0,2 bis 5,0 Gew.-% sauerstoffhaltige Verbindungen des Zinns, berechnet als SnO, enthält. In der Beschreibung wird allgemein die Umsetzung von 2-Hydroxypropylamin und $NH_3$ beschreiben (Seite 25, Zeile 25).

CN 104693037 A (Dalian Institute of Chemical Physics) beschreibt die Umsetzung von Isopropanolamin und Ammoniak in Gegenwart von Wasserstoff bei einem Druck von 20 bis 250 bar und einer Temperatur von 120 bis 230 °C an einem geträgerten Katalysator, der Ni und/oder Cu sowie Promotern ausgewählt aus der Gruppe Fe, Cu, Ru, Re, K, Zn und B enthält, wobei als Träger $SiO_2$ und/oder $Al_2O_3$ eingesetzt werden.

DMDETA lässt sich beispielsweise durch Umsetzung von 1,2-PDA und Methylaziridin in einer Ausbeute von bis zu 24% herstellen - beschrieben in US 2011/151615 A1 (President and Fellows of Harvard College). Ebenfalls möglich ist die Umsetzung von 1,2-Dichlorpropan mit Ammoniak (K. V. Chernitskii, V. A. Bobylev, J. Gen. Chem. USSR, 1990, 60, 1636-1643). Des Weiteren ist es theoretisch möglich, dass DMDETA bei der Herstellung von 1,2-PDA als sogenanntes Koppelprodukt (Wertprodukt) anfällt. Dabei reagiert bereits gebildetes 1,2-PDA unter Abspaltung von Wasser mit nicht umgesetztem MIPOA zum DMDETA. Allerdings ist es oft nicht möglich, DMDETA in ausreichenden Mengen zu erhalten, da es unter Abspaltung von Ammoniak zum Dimethylpiperazin cyclisiert. Im Stand der Technik ist die Bildung des Wertproduktes DMDETA daher nicht beschrieben.

Der vorliegenden Erfindung lag die Aufgabe zugrunde einem oder mehreren Nachteilen des Standes der Technik abzuhelfen. Insbesondere sollte ein Verfahren zur Herstellung von 1,2-PDA gefunden werden, bei dem gleichzeitig DMDETA in signifikanten Mengen gebildet wird. Es sollten Bedingungen gefunden werden, die technisch in einfacher Weise und damit kostengünstig herzustellen sind und die es erlauben, dass Verfahren effizient, insbesondere mit hohem Umsatz, hoher Ausbeute, Raum-Zeit-Ausbeute (RZA) und Selektivität bei gleichzeitig hoher mechanischer Stabilität des Katalysatorformkörpers durchzuführen. Darüber hinaus sollten Wege gefunden werden, 1,2-PDA und DMDETA mit möglichst hoher Reinheit zu erhalten.

[Raum-Zeit-Ausbeuten werden angegeben in 'Produktmenge / (Katalysatorvolumen • Zeit)' (kg/($l_{Kat.}$ • h)) und/oder 'Produktmenge / (Reaktorvolumen • Zeit)' (kg/($l_{Reaktor}$ • h).].

Überraschenderweise wurde ein Verfahren zur kontinuierlichen Herstellung von 1,2-Propylendiamin (1,2-PDA) und Dimethyldiethylentriamin (DMDETA) durch Umsetzung von Monoisopropanolamin (MIPOA) mit Ammoniak in Gegenwart von Wasserstoff und eines geträgerten Heterogen-Hydrierkatalysators (Katalysator) gefunden, das dadurch gekennzeichnet ist, dass die Umsetzung in der Flüssigphase bei einem Absolutdruck im Bereich von 60 bis 170 bar erfolgt.

BESCHREIBUNG DER ERFINDUNG

Es können beispielsweise Katalysatoren eingesetzt werden, deren katalytisch aktive Masse vor deren Reduktion mit Wasserstoff sauerstoffhaltige Verbindungen des Aluminiums und sauerstoffhaltige Verbindungen des Kupfers, Nickels und/oder Kobalts enthält.

Bevorzugt sind Katalysatoren, deren katalytisch aktive Masse vor deren Reduktion mit Wasserstoff sauerstoffhaltige Verbindungen des Aluminiums, Kupfers, Nickels und Kobalts und im Bereich von 0,2 bis 5,0 Gew.-% sauerstoffhaltige Verbindungen des Zinns, berechnet als SnO, enthält.

Insbesondere werden Katalysatoren, deren katalytisch aktive Masse vor deren Reduktion mit Wasserstoff im Bereich von

15 bis 80 Gew.-% sauerstoffhaltige Verbindungen des Aluminiums, berechnet als $Al_2O_3$,
1 bis 20 Gew.-% sauerstoffhaltige Verbindungen des Kupfers, berechnet als CuO,
5 bis 35 Gew.-% sauerstoffhaltige Verbindungen des Nickels, berechnet als NiO,
5 bis 35 Gew.-% sauerstoffhaltige Verbindungen des Kobalts, berechnet als CoO, und
0,2 bis 5,0 Gew.-% sauerstoffhaltige Verbindungen des Zinns, berechnet als SnO, enthält, im oben genannten Aminierungsverfahren eingesetzt.

Bevorzugte Reaktoren sind Rohrreaktoren. Beispiele für geeignete Reaktoren mit Kreisgasstrom finden sich in Ullmann's Encyclopedia of Industrial Chemistry, 5th Ed., Vol. B 4, Seiten 199-238, "Fixed-Bed Reactors".

Alternativ erfolgt die Umsetzung vorteilhaft in einem Rohrbündelreaktor oder in einer Monostranganlage. Bei einer Monostranganlage kann der Rohrreaktor, in dem die Umsetzung erfolgt, aus einer Hintereinanderschaltung mehrerer (z. B. zweier oder dreier) einzelner Rohrreaktoren bestehen. Optional ist hier vorteilhaft eine Zwischeneinspeisung von Feed (enthaltend MIPOA und/oder Ammoniak und/oder $H_2$) und/oder Kreisgas und/oder Reaktoraustrag aus einem nachgeschalteten Reaktor möglich.

Die Kreisgasmenge liegt, im Fall eines $H_2$-Gehalts von 80 Vol.%, bevorzugt im Bereich von 10 bis 450 $Nm^3$ / [$m^3$ Katalysator (Schüttvolumen) • h], insbesondere im Bereich von 15 bis 450 $Nm^3$ / [$m^3$ Katalysator (Schüttvolumen) • h], weiter besonders im Bereich von 20 bis 400 $Nm^3$ / [$m^3$ Katalysator (Schüttvolumen) • h], ganz besonders im Bereich von 25 bis 400 $Nm^3$ / [$m^3$ Katalysator (Schüttvolumen) • h], z.B. 35 bis 360 $Nm^3$ / [$m^3$ Katalysator (Schüttvolumen) • h].

Bei einem anderen $H_2$-Gehalt ändern sich die o.g. Kreisgasmengen rechnerisch entsprechend, um die $H_2$-Menge (in $Nm^3$ / [$m^3$ Katalysator (Schüttvolumen) • h]) konstant zu halten.

[$Nm^3$ = Normkubikmeter = auf Normalbedingungen (20 °C, 1 bar abs.) umgerechnetes Volumen]. (Normaldruck = 1 bar abs.).

Im erfindungsgemäßen Verfahren werden die Katalysatoren bevorzugt in Form von Katalysatoren eingesetzt, die nur aus katalytisch aktiver Masse und gegebenenfalls einem Verformungshilfsmittel (wie z. B. Graphit oder Stearinsäure), falls der Katalysator als Formkörper eingesetzt wird, bestehen, also keine weiteren katalytisch aktiven Begleitstoffe enthalten.

In diesem Zusammenhang wird das oxidische Trägermaterial Aluminiumoxid ($Al_2O_3$) als zur katalytisch aktiven Masse gehörig gewertet.

Die Katalysatoren werden dergestalt eingesetzt, dass man die katalytisch aktive, zu Pulver vermahlene Masse in das Reaktionsgefäß einbringt oder, dass man die katalytisch aktive Masse nach Mahlung, Vermischung mit Formhilfsmitteln, Formung und Temperung als Katalysatorformkörper - beispielsweise als Tabletten, Kugeln, Ringe, Extrudate (z. B. Stränge) - im Reaktor anordnet.

Die Konzentrationsangaben (in Gew.-%) der Komponenten des Katalysators beziehen sich jeweils - falls nicht anders angegeben - auf die katalytisch aktive Masse des fertigen Katalysators nach dessen letzter Wärmebehandlung und vor dessen Reduktion mit Wasserstoff.

Die katalytisch aktive Masse des Katalysators, nach dessen letzter Wärmebehandlung und vor dessen Reduktion mit Wasserstoff, ist als die Summe der Massen der katalytisch aktiven Bestandteile und der o. g. Katalysatorträgermaterialien definiert und enthält im Wesentlichen die folgenden Bestandteile:
Aluminiumoxid ($Al_2O_3$), sauerstoffhaltige Verbindungen des Kupfers, Nickels und Kobalts und sauerstoffhaltige Verbindungen des Zinns.

Die Summe der o. g. Bestandteile der katalytisch aktiven Masse beträgt üblicherweise 70 bis 100 Gew.-%, bevorzugt 80 bis 100 Gew.-%, besonders bevorzugt 90 bis 100 Gew.-%, besonders > 95 Gew.-%, ganz besonders > 98 Gew.-%, insbesondere > 99 Gew.-%, z. B. besonders bevorzugt 100 Gew.-%.

Die katalytisch aktive Masse der im erfindungsgemäßen Verfahren eingesetzten Katalysatoren kann weiterhin ein oder mehrere Elemente (Oxidationsstufe 0) oder deren anorganische oder organische Verbindungen, ausgewählt aus den Gruppen I A bis VI A und I B bis VII B und VIII des Periodensystems, enthalten.

Beispiele für solche Elemente bzw. deren Verbindungen sind:
Übergangsmetalle, wie Mn bzw. $MnO_2$, W bzw. Wolframoxide, Ta bzw. Tantaloxide, Nb bzw. Nioboxide oder Nioboxalat, V bzw. Vanadiumoxide bzw. Vanadylpyrophosphat; Lanthanide, wie Ce bzw. $CeO_2$ oder Pr bzw. $Pr_2O_3$; Erdalkalimetalloxide, wie SrO; Erdalkalimetallcarbonate, wie $MgCO_3$, $CaCO_3$ und $BaCO_3$; Boroxid ($B_2O_3$).

Bevorzugt enthält die katalytisch aktive Masse der im erfindungsgemäßen Verfahren eingesetzten Katalysatoren kein Rhenium, kein Ruthenium, kein Eisen und/oder kein Zink, jeweils weder in metallischer (Oxidationsstufe = 0) noch in einer ionischen (Oxidationsstufe $\neq$ 0), insbesondere oxidierten, Form.

Bevorzugt enthält die katalytisch aktive Masse der im erfindungsgemäßen Verfahren eingesetzten Katalysatoren kein Silber und/oder Molybdän, jeweils weder in metallischer (Oxidationsstufe = 0) noch in einer ionischen (Oxidationsstufe $\neq$ 0), insbesondere oxidierten, Form.

In einer besonders bevorzugten Ausführungsform enthält die katalytisch aktive Masse der im erfindungsgemäßen Verfahren eingesetzten Katalysatoren keine weitere katalytisch aktive Komponente, weder in elementarer (Oxidationsstufe = 0) noch in ionischer (Oxidationsstufe $\neq$ 0) Form.

In der besonders bevorzugten Ausführungsform ist die katalytisch aktive Masse nicht mit weiteren Metallen oder Metallverbindungen dotiert.

Bevorzugt sind jedoch aus der Metallgewinnung von Cu, Co, Ni, Sn herrührende übliche Begleit-Spurenelemente hiervon ausgenommen.

Bevorzugt enthält die katalytisch aktive Masse des Katalysators keine sauerstoffhaltigen Verbindungen des Siliziums und/oder des Zirkoniums.

Bevorzugt enthält die katalytisch aktive Masse des Katalysators keine sauerstoffhaltigen Verbindungen des Titans und/oder des Chroms.

Die katalytisch aktive Masse des Katalysators enthält vor dessen Reduktion mit Wasserstoff im Bereich von 0,2 bis 5,0 Gew.-%, besonders im Bereich von 0,4 bis 4,0 Gew.-%, weiter besonders im Bereich von 0,6 bis 3,0 Gew.-%, weiter besonders bevorzugt im Bereich von 0,7 bis 2,5 Gew.-%, sauerstoffhaltige Verbindungen des Zinns, berechnet als SnO.

Die katalytisch aktive Masse des Katalysators enthält vor dessen Reduktion mit Wasserstoff bevorzugt im Bereich von 5,0 bis 35 Gew.-%, besonders im Bereich von 10 bis 30 Gew.-%, weiter besonders im Bereich von 12 bis 28 Gew.-%, ganz besonders 15 bis 25 Gew.-%, sauerstoffhaltige Verbindungen des Kobalts, berechnet als CoO.

Die katalytisch aktive Masse des Katalysators enthält vor dessen Reduktion mit Wasserstoff weiterhin bevorzugt im Bereich von

15 bis 80 Gew.-%, besonders 30 bis 70 Gew.-%, weiter besonders 35 bis 65 Gew.-%, sauerstoffhaltige Verbindungen des Aluminiums, jeweils berechnet als $Al_2O_3$,
1 bis 20 Gew.-%, besonders 2 bis 18 Gew.-%, weiter besonders 5 bis 15 Gew.-%, sauerstoffhaltige Verbindungen des Kupfers, jeweils berechnet als CuO, und
5 bis 35 Gew.-%, besonders 10 bis 30 Gew.-%, weiter besonders 12 bis 28 Gew.-%, ganz besonders 15 bis 25 Gew.-%, sauerstoffhaltige Verbindungen des Nickels, jeweils berechnet als NiO.

Das Molverhältnis von Nickel zu Kupfer beträgt bevorzugt größer 1, besonders bevorzugt größer 1,2, weiter besonders bevorzugt im Bereich von 1,8 bis 8,5.

Die BET-Oberfläche (ISO 9277:1995) der im erfindungsgemäßen Verfahren eingesetzten Katalysatoren liegt bevorzugt im Bereich von 30 bis 250 $m^2/g$, besonders im Bereich von 90 bis 200 $m^2/g$, weiter besonders im Bereich von 130 bis 190 $m^2/g$, (jeweils vor der Reduktion mit Wasserstoff). Diese Bereiche werden insbesondere durch Calciniertemperaturen bei der Katalysatorherstellung im Bereich von 400 bis 600 °C, besonders 420 bis 550 °C, (vgl. unten) erzielt.

Zur Herstellung der im erfindungsgemäßen Verfahren verwendeten Katalysatoren sind verschiedene Verfahren möglich. Sie sind beispielsweise durch Peptisieren pulvriger Mischungen der Hydroxide, Carbonate, Oxide und/oder anderer Salze der Komponenten mit Wasser und nachfolgendes Extrudieren und Tempern (Wärmebehandlung) der so

erhaltenen Masse erhältlich.

Bevorzugt werden zur Herstellung der Katalysatoren Fällungsmethoden angewandt. So können sie beispielsweise durch eine gemeinsame Fällung der Nickel-, Kobalt-, Kupfer- und Sn-Komponenten aus einer diese Elemente enthaltenden, wässrigen Salzlösung mittels Basen in Gegenwart einer Aufschlämmung einer schwerlöslichen, sauerstoffhaltigen Aluminiumverbindung und anschließendes Waschen, Trocknen und Calcinieren des erhaltenen Präzipitats erhalten werden. Als schwerlösliche, sauerstoffhaltige Aluminiumverbindungen können beispielsweise Aluminiumoxid, Aluminiumoxidhydrat, Aluminiumphosphate, -borate und - silikate Verwendung finden. Die Aufschlämmungen der schwerlöslichen Aluminiumverbindungen können durch Suspendieren feinkörniger Pulver dieser Verbindungen in Wasser unter kräftigem Rühren hergestellt werden. Vorteilhaft werden diese Aufschlämmungen durch Ausfällen der schwerlöslichen Aluminiumverbindungen aus wässrigen Aluminiumsalzlösungen mittels Basen erhalten.

Bevorzugt werden die Katalysatoren über eine gemeinsame Fällung (Mischfällung) aller ihrer Komponenten hergestellt. Dazu wird zweckmäßigerweise eine die Katalysatorkomponenten enthaltende, wässrige Salzlösung in der Wärme und unter Rühren so lange mit einer wässrigen Base, - beispielsweise Natriumcarbonat, Natriumhydroxid, Kaliumcarbonat oder Kaliumhydroxid - versetzt, bis die Fällung vollständig ist. Es kann auch mit Alkalimetall-freien Basen wie Ammoniak, Ammoniumcarbonat, Ammoniumhydrogencarbonat, Ammoniumcarbamat, Ammoniumoxalat, Ammoniummalonat, Urotropin, Harnstoff, etc. gearbeitet werden. Die Art der verwendeten Salze ist im Allgemeinen nicht kritisch: Da es bei dieser Vorgehensweise vornehmlich auf die Wasserlöslichkeit der Salze ankommt, ist ein Kriterium ihre zur Herstellung dieser verhältnismäßig stark konzentrierten Salzlösungen erforderliche, gute Wasserlöslichkeit. Es wird als selbstverständlich erachtet, dass bei der Auswahl der Salze der einzelnen Komponenten natürlich nur Salze mit solchen Anionen gewählt werden, die nicht zu Störungen führen, sei es, indem sie unerwünschte Fällungen verursachen oder indem sie durch Komplexbildung die Fällung erschweren oder verhindern.

Die bei diesen Fällungsreaktionen erhaltenen Niederschläge sind im Allgemeinen chemisch uneinheitlich und bestehen u.a. aus Mischungen der Oxide, Oxidhydrate, Hydroxide, Carbonate und unlöslichen und basischen Salze der eingesetzten Metalle. Es kann sich für die Filtrierbarkeit der Niederschläge als günstig erweisen, wenn sie gealtert werden, d. h. wenn man sie noch einige Zeit nach der Fällung, gegebenenfalls in Wärme oder unter Durchleiten von Luft, sich selbst überlässt.

Die nach diesen Fällungsverfahren erhaltenen Niederschläge werden zu den Katalysatoren wie üblich weiterverarbeitet. Zunächst werden die Niederschläge gewaschen. Über die Dauer des Waschvorgangs und über die Temperatur und Menge des Waschwassers kann der Gehalt an Alkalimetall, das durch die als Fällungsmittel eventuell verwendete (Mineral)base zugeführt wurde, beeinflusst werden. Im Allgemeinen wird durch Verlängerung der Waschzeit oder Erhöhung der Temperatur des Waschwassers der Gehalt an Alkalimetall abnehmen. Nach dem Waschen wird das Fällgut im Allgemeinen bei 80 bis 200 °C, vorzugsweise bei 100 bis 150 °C, getrocknet und danach calciniert. Die Calcinierung wird im Allgemeinen bei Temperaturen zwischen 300 und 800 °C, vorzugsweise bei 400 bis 600 °C, insbesondere bei 420 bis 550 °C ausgeführt.

Die Katalysatoren können auch durch Tränkung von Aluminiumoxid ($Al_2O_3$), das beispielsweise in Form von Pulver oder Formkörpern, wie Strängen, Tabletten, Kugeln oder Ringen, vorliegt, hergestellt werden.

Das Aluminiumoxid wird beispielsweise in der amorphen, gamma-, theta- und/oder delta- Form, als Aluminiumoxohydroxid (Böhmit), bevorzugt in der amorphen Form eingesetzt.

Die Herstellung von Formkörpern kann nach den üblichen Verfahren erfolgen.

Die Tränkung erfolgt ebenfalls nach den üblichen Verfahren, wie z. B. in A. B. Stiles, Catalyst Manufacture - Laboratory and Commercial Preparations, Marcel Dekker, New York (1983) beschrieben, durch Aufbringung einer jeweils entsprechenden Metallsalzlösung in einer oder mehreren Tränkstufen, wobei als Metallsalze z. B. entsprechende Nitrate, Acetate oder Chloride verwendet werden. Die Masse wird im Anschluss an die Tränkung getrocknet und optional calciniert.

Die Tränkung kann nach der sogenannten "incipient wetness"-Methode erfolgen, bei der das Aluminiumoxid entsprechend seiner Wasseraufnahmekapazität maximal bis zur Sättigung mit der Tränklösung befeuchtet wird. Die Tränkung kann aber auch in überstehender Lösung erfolgen.

Bei mehrstufigen Tränkverfahren ist es zweckmäßig, zwischen einzelnen Tränkschritten zu trocknen und optional zu calcinieren. Die mehrstufige Tränkung ist vorteilhaft besonders dann anzuwenden, wenn das Aluminiumoxid mit einer größeren Metallmenge beaufschlagt werden soll.

Zur Aufbringung der Metallkomponenten auf das Aluminiumoxid kann die Tränkung gleichzeitig mit allen Metallsalzen oder in beliebiger Reihenfolge der einzelnen Metallsalze nacheinander erfolgen.

Anschließend werden die durch Tränkung hergestellten Katalysatoren getrocknet und bevorzugt auch calciniert, z. B. bei den bereits oben angegebenen Calciniertemperaturbereichen.

Nach der Calcinierung wird der Katalysator zweckmäßigerweise konditioniert, sei es, dass man ihn durch Vermahlen auf eine bestimmte Korngröße einstellt oder dass man ihn nach seiner Vermahlung mit Formhilfsmitteln wie Graphit oder Stearinsäure vermischt, mittels einer Presse zu Formlingen, z. B. Tabletten, verpresst und tempert. Die Tempertemperaturen entsprechen dabei bevorzugt den Temperaturen bei der Calcinierung.

Die auf diese Weise hergestellten Katalysatoren enthalten die katalytisch aktiven Metalle in Form eines Gemisches ihrer sauerstoffhaltigen Verbindungen, d. h. insbesondere als Oxide und Mischoxide.

Die z. B. wie oben beschrieben hergestellten Katalysatoren werden als solche gelagert und ggf. gehandelt. Vor ihrem Einsatz als Katalysatoren werden sie üblicherweise vorreduziert. Sie können jedoch auch ohne Vorreduktion eingesetzt werden, wobei sie dann unter den Bedingungen der hydrierenden Aminierung durch den im Reaktor vorhandenen Wasserstoff reduziert werden.

Zur Vorreduktion werden die Katalysatoren zunächst bei bevorzugt 150 bis 200 °C über einen Zeitraum von z. B. 12 bis 20 Stunden einer Stickstoff-Wasserstoff-Atmosphäre ausgesetzt und anschließend noch bis zu ca. 24 Stunden bei bevorzugt 200 bis 400 °C in einer Wasserstoffatmosphäre behandelt. Bei dieser Vorreduktion wird ein Teil der in den Katalysatoren vorliegenden sauerstoffhaltigen Metallverbindungen zu den entsprechenden Metallen reduziert, so dass diese gemeinsam mit den verschiedenartigen Sauerstoffverbindungen in der aktiven Form des Katalysators vorliegen.

Das erfindungsgemäße Verfahren wird kontinuierlich durchgeführt, wobei der Katalysator bevorzugt als Festbett im Reaktor angeordnet ist. Dabei ist sowohl eine Anströmung des Katalysatorfestbetts von oben als auch von unten möglich.

Als Edukt wird MIPOA eingesetzt. Es gibt zwei unterschiedliche Konstitutionsisomere des MIPOA; 1-Aminopropan-2-ol und 2-Aminopropan-1-ol. Beide Isomere können erfindungsgemäß zum 1,2-PDA umgesetzt werden. Es können also 1-Aminopropan-2-ol, 2-Aminopropan-1-ol oder ein Gemisch dieser beiden Isomere eingesetzt werden. Wird ein solches Gemisch eingesetzt, so meint eine Bezugnahme auf MIPOA in dieser Anmeldung immer beide Isomere. Bevorzugt wird MIPOA eingesetzt, dass durch Umsetzung von Propylenoxid mit Ammoniak hergestellt wurde. Dabei entstehen überwiegend 1-Aminopropan-2-ol, aber auch geringe Mengen an 2-Aminopropan-1-ol, dessen Anteil üblicherweise 2 bis 10 Gew.-%, insbesondere 4 bis 6 Gew.-%, bezogen auf die Gesamtmasse der beiden Isomere, beträgt.

Das erfindungsgemäß hergestellte DMDETA fällt in drei unterschiedlichen Konstitutsionsisomere an. Dabei handelt es sich um N2-(2-Aminopropyl)propan-1,2-diamin, N1-(2-Aminopropyl)propan-1,2-diamin und N2-(2-Amino-1-methyl-ethyl)propan-1,2-diamin.

Der Ammoniak wird üblicherweise in der 5- bis 30-fachen molaren Menge, bevorzugt 6- bis 29-fachen molaren Menge, weiter bevorzugt 7- bis 28-fachen molaren Menge, besonders 8- bis 27-fachen molaren Menge, insbesondere in der 9- bis 26-fachen molaren Menge, in der 9- bis 25-fachen molaren Menge, der 9- bis 20-fachen molaren Menge, oder der 9- bis 15-fachen molaren Menge, jeweils bezogen auf das eingesetzte MIPOA, eingesetzt.

Der Ammoniak wird bevorzugt ohne ein weiteres Lösungsmittel (Druckgas, Reinheit besonders 95 bis 100 Gew.-%ig) eingesetzt.

Das Edukt MIPOA wird bevorzugt in einer Reinheit von 95 bis 100 Gew.-%, besonders 98 bis 100 Gew.-% eingesetzt.

Bevorzugt wird eine Abgasmenge von 1 bis 450 Normkubikmeter / ($m^3$ Katalysator (Schüttvolumen) • h), insbesondere 2 bis 200 Normkubikmeter / ($m^3$ Katalysator (Schüttvolumen) • h), gefahren. [Normkubikmeter = auf Normalbedingungen (20 °C, 1 bar abs.) umgerechnetes Volumen]. Katalysatorvolumen-Angaben beziehen sich immer auf das Schüttvolumen.

Die Aminierung der Alkoholgruppe des Edukts MIPOA wird in der Flüssigphase durchgeführt. Bevorzugt ist das Festbettverfahren.

Beim Arbeiten in der Flüssigphase leitet man die Edukte (MIPOA, Ammoniak), bevorzugt simultan, in flüssiger Phase bei Drücken von 60 bis 170 bar, bevorzugt 70 bis 145 bar, weiter bevorzugt 80 bis 140 bar, weiter bevorzugt 90 bis 135 bar, besonders bevorzugt 100 bis 130 bar, z.B. 110 bis 125 bar und Temperaturen von 120 bis 240 °C, besonders 130 bis 230 °C, bevorzugt 140 bis 220 °C, insbesondere 150 bis 210 °C, weiter besonders 160 bis 200 °C, weiter ganz besonders 162 bis 190 °C, z.B. 165 bis 180 °C, inklusive Wasserstoff über den Katalysator, der sich üblicherweise in einem bevorzugt von außen beheizten Festbettreaktor befindet.

Die Temperatur wird so gewählt, dass bei einem gegebenen Druck ein bestimmter Umsatz von MIPOA erfolgt. Üblicherweise wird die Reaktion bei Umsätzen von 70 bis 100%, bevorzugt 80 bis 99,9%, ebenfalls bevorzugt 82 bis 99,5% gefahren. Der Umsatz berechnet sich wie folgt:

$$U = (1 - \frac{n}{n_0}) \cdot 100\%$$

wobei n die Reststoffmenge MIPOA nach erfolgter Umsetzung und $n_0$ die ursprüngliche Stoffmenge von MIPOA vor Beginn der Umsetzung bedeuten. Die Stoffmengen lassen sich mittels Gaschromatographie, so wie im Beispielteil dieser Anmeldung beschrieben, bestimmten.

Beim Arbeiten in der Flüssigphase ist sowohl eine Rieselfahrweise als auch eine Sumpffahrweise möglich. Die Sumpffahrweise ist bevorzugt. Die Katalysatorbelastung liegt im Allgemeinen im Bereich von 0,1 bis 0,7, bevorzugt 0,15

bis 0,6, besonders bevorzugt 0,2 bis 0,5, kg MIPOA pro Liter Katalysator (Schüttvolumen) und Stunde (MIPOA berechnet als 100 Gew.-%ig). Gegebenenfalls kann eine Verdünnung der Edukte mit einem geeigneten Lösungsmittel, wie Wasser, Tetrahydrofuran, Dioxan, N-Methylpyrrolidon oder Ethylenglykoldimethylether, erfolgen. Bevorzugt erfolgt keine Verdünnung der Edukte mit einem Lösungsmittel, d.h. die erfindungsgemäße Umsetzung erfolgt lösungsmittelfrei. Es ist zweckmäßig, die Reaktanden bereits vor der Zuführung in das Reaktionsgefäß zu erwärmen, und zwar bevorzugt auf die Reaktionstemperatur.

Die Umsetzung wird in Gegenwart von 1,0 bis 4,5 Gew.-% Wasserstoff, besonders in Gegenwart von 1,2 bis 4,5 Gew.-% Wasserstoff, weiter besonders in Gegenwart von 1,5 bis 4,0 Gew.-% Wasserstoff, ganz besonders in Gegenwart von 2,0 bis 4,0 Gew.-% Wasserstoff, weiter ganz besonders in Gegenwart von 2,5 bis 3,7 Gew.-% Wasserstoff, jeweils bezogen auf die Menge an eingesetztem MIPOA, durchgeführt.

Der Druck im Reaktionsgefäß, welcher sich aus der Summe der Partialdrücke des Ammoniaks, des MIPOAs und der gebildeten Reaktionsprodukte sowie ggf. des mitverwendeten Lösungsmittels bei den angegebenen Temperaturen ergibt, wird zweckmäßigerweise durch Aufpressen von Wasserstoff auf den gewünschten Reaktionsdruck erhöht (Absolutdruck). Wenn nicht explizit etwas anderes angegeben ist, beziehen sich alle hierin genannten Druckangaben auf den Absolutdruck.

Ferner kann der überschüssige Ammoniak zusammen mit dem Wasserstoff im Kreis geführt werden.

Ist der Katalysator als Festbett angeordnet, kann es für die Selektivität der Reaktion vorteilhaft sein, die Katalysatorformkörper im Reaktor mit inerten Füllkörpern zu vermischen, sie sozusagen zu "verdünnen". Der Anteil der Füllkörper in solchen Katalysatorzubereitungen kann 20 bis 80, besonders 30 bis 60 und insbesonders 40 bis 50 Volumenteile betragen.

Das im Zuge der Umsetzung gebildete Reaktionswasser (jeweils ein Mol pro Mol umgesetzte

Alkoholgruppe) wirkt sich im Allgemeinen auf den Umsetzungsgrad, die Reaktionsgeschwindigkeit, die Selektivität und die Katalysatorstandzeit nicht störend aus und wird deshalb zweckmäßigerweise erst bei der Aufarbeitung des Reaktionsproduktes aus diesem entfernt, z. B. destillativ.

Das Produkt aus der erfindungsgemäßen Umsetzung kann weiter aufgearbeitet werden. Erfindungsgemäß erfolgt die Umsetzung in einem Reaktor, dessen Austrag (nachdem er ggf. zweckmäßig entspannt wurde, um überschüssigen Wasserstoff abzutrennen) destillativ aufgetrennt wird, wobei

(1) der Reaktoraustrag einer ersten Destillationskolonne K1 zugeführt wird, in der Ammoniak an einem Seitenabzug oder über Kopf abgetrennt wird,
(2) der Sumpfaustrag aus K1 einer zweite Destillationskolonne K2 zugeführt wird, in der Wasser an einem Seitenabzug oder über Kopf abgetrennt wird,
(3) der Sumpfaustrag aus K2 einer dritten Destillationskolonne K3 zugeführt wird, in der 1,2-PDA an einem Seitenabzug oder über Kopf abgetrennt wird,
(4) der Sumpfaustrag aus K3 einer vierten Destillationskolonne K4 zugeführt wird, in der DMDETA im Sumpf, MIPOA an einem Seitenabzug und Leichtsieder über Kopf abgetrennt werden.

Bevorzugt werden der Ammoniak, das Wasser sowie 1,2-PDA jeweils über Kopf abgetrennt.

Das Edukt MIPOA wird bevorzugt gasförmig abgetrennt. Bevorzugt befindet sich der Seitenabzug im Abtriebsteil von K4.

In K4 werden üblicherweise Leichtsieder (beispielsweise Piperazine, insbesondere Dimethylpiperazin) über Kopf abgetrennt. Als Leichtsieder werden solche organischen Komponenten bezeichnet, die unter den gegebene Destillationsbedingungen (Druck und Temperatur) einen geringeren Siedepunkt als 2-Aminopropan-1-ol oder 1-Aminopropan-2-ol, je nachdem welches der beiden unter den gegeben Destillationsbedingungen den geringeren Siedepunkt hat, besitzen. Insbesondere sind Leichtsieder organische Komponenten, die einen Siedepunkt von < 159 °C (1,013 bar) besitzen.

Der in Schritt (1) erhaltene Ammoniak und/oder das in Schritt (4) erhaltene MIPOA kann in den Reaktor zurückgeführt werden. Bevorzugt werden beide zurückgeführt.

In Schritt (1) abgetrennter Ammoniak wird besonders mit einer Reinheit von 90 bis 99,9 Gew.-%, weiter besonders 95 bis 99,9 Gew.-%, in die Umsetzung zurückgeführt, wobei in einer besonderen Ausgestaltung ein Teil des abgetrennten Ammoniaks, besonders 1 bis 30 Gew.-% des abgetrennten Ammoniaks, weiter besonders 2 bis 20 Gew.-% des abgetrennten Ammoniaks, ausgeschleust wird.

Das in Schritt (3) erhaltenen 1,2-PDA hat üblicherweise eine Reinheit von 90 bis 99,98 Gew.-%, insbesondere 95 bis 99,9 Gew.-%.

In Schritt (4) abgetrenntes MIPOA wird besonders mit einer Reinheit von 90 bis 99,9 Gew.-%, weiter besonders 95 bis 98 Gew.-%, in die Umsetzung zurückgeführt.

In Schritt (4) abgetrenntes DMDETA hat üblicherweise eine Reinheit von 90 bis 99,9 Gew.-%, insbesondere 94 bis 97,5 Gew.-%.

Der Kopfdruck in K1 beträgt bevorzugt 10 bis 25 bar, besonders bevorzugt 15 bis 20 bar und ganz besonders bevorzugt 17 bis 19 bar. Die Kopftemperatur beträgt bevorzugt 20 bis 70 °C, besonders bevorzugt 30 bis 60 °C und ganz besonders bevorzugt 40 bis 50 °C. Die Sumpftemperatur beträgt bevorzugt 180 bis 250 °C, besonders bevorzugt 190 bis 240 °C und ganz besonders bevorzugt 200 bis 230 °C.

Der Kopfdruck in K2 beträgt bevorzugt 0,3 bis 2,5 bar, besonders bevorzugt 0,5 bis 2 bar und ganz besonders bevorzugt 1 bis 1,5 bar. Die Kopftemperatur beträgt bevorzugt 80 bis 150 °C, besonders bevorzugt 90 bis 130 °C und ganz besonders bevorzugt 100 bis 120 °C. Die Sumpftemperatur beträgt bevorzugt 100 bis 200 °C, besonders bevorzugt 110 bis 160 °C und ganz besonders bevorzugt 120 bis 150 °C.

Der Kopfdruck in K3 beträgt bevorzugt 0,1 bis 3 bar, besonders bevorzugt 0,4 bis 2 bar und ganz besonders bevorzugt 0,5 bis 1,5 bar. Die Kopftemperatur beträgt bevorzugt 20 bis 200 °C, besonders bevorzugt 50 bis 190 °C und ganz besonders bevorzugt 80 bis 150 °C. Die Sumpftemperatur beträgt bevorzugt 100 bis 300 °C, besonders bevorzugt 120 bis 250 °C und ganz besonders bevorzugt 130 bis 200 °C.

Der Kopfdruck in K4 beträgt bevorzugt 0,1 bis 3 bar, besonders bevorzugt 0,3 bis 1,5 bar und ganz besonders bevorzugt 0,5 bis 1 bar. Die Kopftemperatur beträgt bevorzugt 80 bis 200 °C, besonders bevorzugt 100 bis 190 °C und ganz besonders bevorzugt 120 bis 170 °C. Die Sumpftemperatur beträgt bevorzugt 100 bis 300 °C, besonders bevorzugt 150 bis 250 °C und ganz besonders bevorzugt 190 bis 220 °C.

In einer weiteren Destillationsstufe (Schritt (5)) kann das im Sumpf der Destillationskolonne K4 abgetrennte DMDETA einer fünften Destillationskolonne K5 zugeführt werden, in der DMDETA mit einer verringerten APHA-Farbzahl über Kopf abgetrennt wird. Bevorzugt erfolgt die Destillation in K5 in Gegenwart eines Zusatzes zur Verbesserung der APHA-Farbzahl, bspw. $NaBH_4$ oder Phosphonsäure.

Das so hergestellte DMDETA hat üblicherweise eine APHA-Farbzahl von $\leq$ 20, besonders $\leq$ 15, ganz besonders $\leq$ 10, z.B. 2 bis 8.

APHA-Farbzahlen werden bestimmt gemäß DIN EN 1557.

Als Destillationskolonnen K1 bis K5 kommen alle möglichen dem Fachmann bekannten Kolonnentypen in Frage. Bevorzugt sind Packungskolonnen mit strukturierten Packungen oder Füllkörpern sowie Bodenkolonnen mit Böden wie Siebböden, Glockenböden oder Ventilböden.

Im Stand der Technik ist kein solches Destillationsverfahren zur Herstellung von 1,2-PDA und DMDETA beschrieben. Es ergeben sich insbesondere die folgenden, für den Fachmann überraschenden Vorteile. Mithilfe der vierten Destillationskolonnen K4 lässt sich DMDETA mit hoher Reinheit herstellen. DMDETA, das die o.g. Reinheit aufweist, und als Koppelprodukt bei der Herstellung von 1,2-PDA entsteht, ist im Stand der Technik nirgendwo beschrieben. Weitere überraschende Vorteile ergeben sich hinsichtlich der Rückführung von MIPOA. So wurde gefunden, dass keine Aufpegelung von Nebenprodukten (bspw. Methyl- oder Dimethylpiperazinen) erfolgt. Es ist daher nicht notwendig, das MIPOA, welches bevorzugt in der oben genannten Reinheit vorliegt, teilweise auszuschleusen. Dies stellt einen erheblichen wirtschaftlichen Vorteil dar, weil das abgetrennte MIPOA so vollständig in die Umsetzung zurückgeführt werden kann.

Abbildung 1 zeigt eine besondere Ausführungsform des erfindungsgemäßen Verfahrens, bei dem 1,2-PDA und DMDETA durch eine 4-Kolonnenverschaltung gewonnen werden.

Die folgenden Beispiele dienen der Verdeutlichung der Erfindung, ohne sie in irgendeiner Weise zu beschränken.

BEISPIELE

Die Herstellung des Katalysators erfolgt gemäß Beispiel B3 nach WO2013/072289 A1.

Beschreibung der Experimente:

Ergebnisse:

Die Ergebnisse sind in den Tabellen 1 und 2 für unterschiedliche Molverhältnisse von Ammoniak zu MIPOA angegeben.

Beispiel 1 (erfindungsgemäß, Tabelle 1, Eintrag 1)

Ein Rohrreaktor wurde mit 47 mL des Katalysators gefüllt. Nach der Aktivierung des Katalysators unter Wasserstoff, wurden bei 120 bar und einer Temperatur von 165 °C kontinuierlich 18 g/h einer Mischung von 1-Aminopropan-2-ol und 2-Aminopropan-1-ol (95:5), 42 g/h Ammoniak (Molverhältnis 10) und 6 NL/h Wasserstoff (2,9 Gew.-% bezogen MIPOA) in den Reaktor geleitet. Nach dem Entspannen des Reaktionsaustrags auf Normaldruck, wurde die Zusammensetzung durch Gaschromatographie analysiert. Der Produktstrom enthielt 78,9% 1,2-PDA und 2,87% DMDETA und

12,2% 1-Aminopropan-2-ol und 2,85% 2-Aminopropan-1-ol (Prozentangaben beziehen sich auf GF-Fl.-%).

Beispiel 2 (nicht-erfindungsgemäß, Tabelle 1, Eintrag 2)

Ein Rohrreaktor wurde mit 47 mL des Katalysators gefüllt. Nach der Aktivierung des Katalysators unter Wasserstoff, wurden bei 120 bar und einer Temperatur von 175 °C kontinuierlich 18 g/h 1-Aminopropan-2-ol und 2-Aminopropan-1-ol (95:5), 42 g/h Ammoniak (Molverhältnis 10) und 6 NL/h Wasserstoff in den Reaktor geleitet. Nach dem Entspannen des Reaktionsaustrags auf Normaldruck, wurde die Zusammensetzung durch Gaschromatographie analysiert. Der Produktstrom enthielt 79,4% 1,2-PDA und 2,61% DMDETA und 12,0% 1-Aminopropan-2-ol und 2,74% 2-Aminopropan-1-ol (Prozentangaben beziehen sich auf GF-Fl.-%).

Die übrigen Versuche wurden analog durchgeführt. Die Ergebnisse sind in den Tabellen 1 und 2 zusammengefasst.

Die GC-Analytik zur Bestimmung des Umsatzes und der Selektivität erfolgte auf einer 30 m RTX-5-Säule. Die Proben wurden bei 50 °C injiziert. Nach fünf Minuten bei dieser Temperatur wurde mit 5 °C/Minute auf 280 °C geheizt, und der Ofen bei dieser Temperatur 5 Minuten gehalten.

Tabelle 1 (Ergebnisse für MV = 10)

| Eintrag | MV | Druck / bar | Temperatur /°C | Umsatz | Sel. (1,2-PDA) | Sel. (DMDETA) | Sel. (1,2-PDA + DMDETA) |
|---|---|---|---|---|---|---|---|
| 1 (erfindungsgemäß) | 10 | 120 | 165 | 85% | 93% | 3% | 96% |
| 2 (nicht-erfindungsgemäß) | 10 | 200 | 175 | 85% | 93% | 3% | 96% |
| 3 (erfindungsgemäß) | 10 | 120 | 180 | 98% | 80% | 6% | 86% |
| 4 (nicht-erfindungsgemäß) | 10 | 200 | 195 | 98% | 77% | 4% | 81% |
| MV = Molare Menge von Ammoniak bezogen auf MIPOA<br>Umsatz: Umsatz bezogen auf MIPOA<br>Sel. (1,2-PDA): Selektivität bezogen auf 1,2-PDA<br>Sel. (DMDETA): Selektivität bezogen auf DMDETA<br>Sel. (1,2-PDA + DMDETA): Selektivität bezogen auf 1,2-PDA und DMDETA | | | | | | | |

Die Temperatur wurde so eingestellt, dass der gewünschte Umsatz von 85 % bzw. 98 % erzielt wurde.

Tabelle 2 (Ergebnisse für MV = 12)

| Eintrag | MV | Druck / bar | Temperatur / °C | Umsatz | Sel. (1,2-PDA) | Sel. (DMDETA) | Sel. (1,2-PDA + DMDETA) |
|---|---|---|---|---|---|---|---|
| 1 (erfindungsgemäß) | 12 | 120 | 165 | 87% | 93% | 3% | 96% |
| 2 (nicht-erfindungsgemäß) | 12 | 200 | 175 | 87% | 93% | 3% | 96% |
| 3 (erfindungsgemäß) | 12 | 120 | 180 | 99% | 82% | 5% | 87% |

(fortgesetzt)

| Eintrag | MV | Druck / bar | Temperatur / °C | Umsatz | Sel. (1,2-PDA) | Sel. (DMDETA) | Sel. (1,2-PDA + DMDETA) |
|---|---|---|---|---|---|---|---|
| 4 (nicht-erfindungsgemäß) | 12 | 200 | 195 | 99% | 77% | 3% | 80% |

MV = Molare Menge von Ammoniak bezogen auf MIPOA
Umsatz: Umsatz bezogen auf MIPOA
Sel. (1,2-PDA): Selektivität bezogen auf 1,2-PDA
Sel. (DMDETA): Selektivität bezogen auf DMDETA
Sel. (1,2-PDA + DMDETA): Selektivität bezogen auf 1,2-PDA und DMDETA

Die Temperatur wurde so eingestellt, dass der gewünschte Umsatz von 87 % bzw. 99 % erzielt wurde.

Diskussion der Ergebnisse:

Die Ergebnisse zeigen, dass bei Umsätzen von 85% bzw. 87% keine Verringerung der Selektivitäten von 1,2-PDA und DMDETA eintritt, wenn der Druck von 200 bar auf 120 bar verringert wird. Bei höheren Umsätzen (98% bzw. 99%) ist sogar eine Steigerung der Selektivitäten von 1,2-PDA und DMDETA zu beobachten, wenn der Druck von 200 bar auf 120 bar verringert wird. Diese Ergebnisse sind für den Fachmann überraschend und weder aus dem Stand der Technik alleine noch in Kombination mit dem allgemeinen Fachwissen ableitbar.

Die Bildung von 1,2-PDA und DMDETA sowie seine (unerwünschte) Cyclisierung zum Dimethylpiperazin lässt sich anhand des folgenden Reaktionsschemas beschreiben:

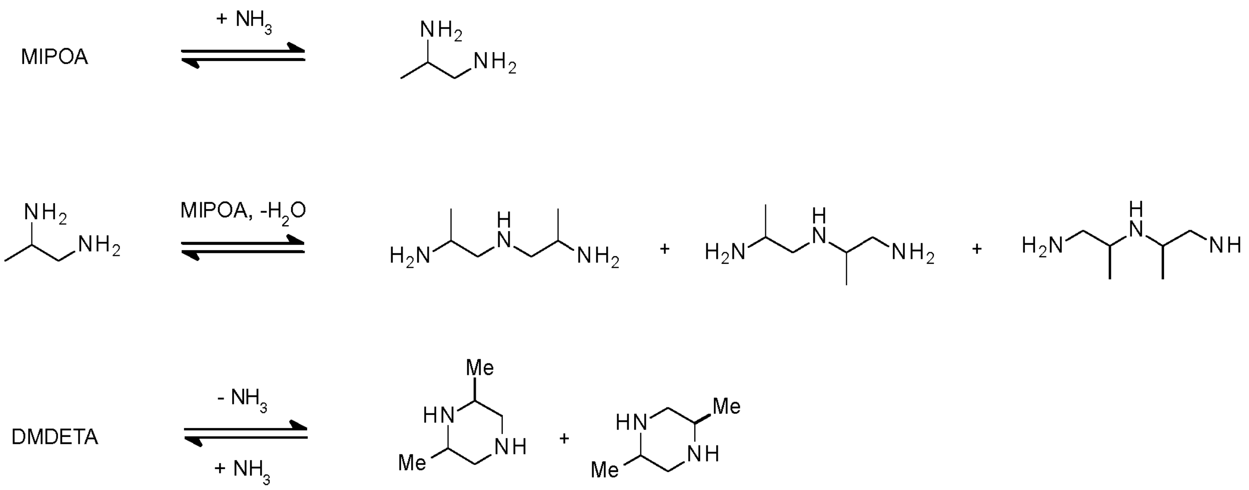


Der Fachmann würde erwarten, dass bei höheren Drücken (200 bar) eine bessere Selektivität bezüglich 1,2-PDA als bei niedrigeren Drücken (120 bar) erzielt wird. Die Umsetzung von 1,2-PDA erfolgt erfindungsgemäß in der Flüssigphase. Die Menge an in der Flüssigphase gelöstem Ammoniak ist umso größer, je höher der Druck ist. Je mehr Ammoniak dort zur Verfügung steht, umso schneller kann das MIPOA mit diesem reagieren. D.h. die Wahrscheinlichkeit, dass (noch) nicht umgesetztes MIPOA in etwaigen Nebenreaktion umgesetzt wird, sinkt. Folglich hätte der Fachmann erwartet, dass auch die Selektivität zum 1,2-PDA bei einer Verringerung des Drucks (von 200 bar auf 120 bar) abnimmt.

Ebenso hätte der Fachmann erwartet, dass bei höheren Drücken (200 bar) eine bessere Selektivität bezüglich DMDETA als bei niedrigeren Drücken (120 bar) erzielt wird. So resultiert die Abnahme der Selektivität zum DMDETA insbesondere daraus, dass dieses unter Abspaltung von Ammoniak zum Dimethylpiperazin cyclisiert. Der Fachmann würde eigentlich erwarten, dass bei höheren Drücken die Bildung von Dimethylpiperazin gemäß dem Prinzip von Le Chatelier verringert ist, da ein höherer Druck der Abspaltung von Ammoniak entgegenwirkt.

Insofern zeigen die in den Tabellen 1 und 2 dargestellten Ergebnisse gerade das Gegenteil dessen, was der Fachmann erwartet hätte und sind mithin überraschend.

Destillative Aufarbeitung

Der flüssige Reaktionsaustrag wird in einem Destillationsteil aufgearbeitet.

Die nachfolgenden Beispiele beruhen auf Simulationsergebnissen, die mit der Software Aspen der Firma Aspen Technology, Inc. erzielt wurden. Die in dem Programm verwendeten thermodynamischen Parameter für die einzelnen Reaktionsprodukte basieren auf veröffentlichten thermodynamischen Daten bzw. eigenen Messungen. Die Spezifizierung und die Simulation der angegebenen, verwendeten Destillationskolonnen erfolgte mit den üblichen, in der Software erhaltenen Routinen.

Zur Optimierung des Simulationsmodells wurden die simulierten Ergebnisse mit experimentellen Ergebnissen, soweit vorhanden, verglichen und das Simulationsmodel an die experimentellen Ergebnisse angepasst, so dass eine gute Übereinstimmung zwischen Simulation und experimentellen Daten erzielt werden konnte.

Die nachfolgenden Beispiele wurden mit dem optimierten Simulationsmodell berechnet.

Die Aufarbeitung erfolgte in vier Kolonnen wie in Abbildung 1 dargestellt.

Ammoniakkolonne (Destillationskolonne K1)

Überschüssiger Ammoniak wird mit Hilfe der Ammoniakkolonne über Kopf abgetrennt und zurück in die Synthesestufe geführt.

Destillationsbedingungen:

| | |
|---|---|
| Kopfdruck: | 18 bar |
| Kopftemperatur: | 45 °C |
| Sumpftemperatur: | 217°C |

Der Sumpf der Ammoniakkolonne enthält noch Wasser, die Wertprodukte 1,2-PDA und DMDETA sowie unumgesetztes MIPOA und wird der Wasserkolonne (Destillationskolonne K2) zugeleitet.

Wasserkolonne (Destillationskolonne K2)

In der Kolonne erfolgt die Abtrennung des Reaktionswassers über Kopf.

Destillationsbedingungen:

| | |
|---|---|
| Kopfdruck: | 1,25 bar |
| Kopftemperatur: | 104 °C |
| Sumpftemperatur: | 135 °C |

Der Sumpf (enthaltend MIPOA, 1,2 PDA, DMDETA und Verunreinigungen) wird der 1,2-PDA-Kolonne (Destillationskolonne K3) zugeführt.

1,2-PDA-Kolonne (Destillationskolonne K3)

In der 1,2-PDA-Kolonne wird 1,2 PDA mit einer Reinheit von ≥ 99,8 Gew.-% über Kopf abgetrennt.

Destillationsbedingungen:

| | |
|---|---|
| Kopfdruck: | 0,95 bar (950 mbar) |
| Kopftemperatur: | 119 °C |
| Sumpftemperatur: | 158 °C |

Der Sumpf (MIPOA, DMDETA und Verunreinigungen) wird der DMDETA-Kolonne (Destillationskolonne K4) zugeführt.

DMDETA-Kolonne (Destillationskolonne K4)

In der Kolonne erfolgt die Aufreinigung des unumgesetzten MIPOAs bevor es wieder der erfindungsgemäßen Umsetzung zugeführt wird. Hierzu wird über Kopf ein Azeotrop aus MIPOA und Methylpiperazinen ausgeschleust.

Destillationsbedingungen:

| | |
|---|---|
| Kopfdruck: | 0,76 bar (760 mbar) |
| Kopftemperatur: | 147 °C |
| Sumpftemperatur: | 202 °C |

Das von Dimethyl-Piperazinen befreite Edukt MIPOA wird gasförmig am Seitenabzug im Abtriebsteil der Kolonne mit einer Reinheit von ≥ 97 Gew.-% erhalten. Im Sumpf wird DMDETA mit einer Reinheit von ≥ 96 Gew.-% erhalten und ausgeschleust.

## Patentansprüche

1. Verfahren zur kontinuierlichen Herstellung von 1,2-Propylendiamin (1,2-PDA) und Dimethyldiethylentriamin (DMDETA) durch Umsetzung von Monoisopropanolamin (MIPOA) mit Ammoniak in Gegenwart von Wasserstoff und eines geträgerten Heterogen-Hydrierkatalysators (Katalysator) **dadurch gekennzeichnet, dass** die Umsetzung in der Flüssigphase bei einem Absolutdruck im Bereich von 60 bis 170 bar in einem Reaktor erfolgt und der Reaktoraustrag destillativ aufgetrennt wird, wobei

   (1) der Reaktoraustrag einer ersten Destillationskolonne K1 zugeführt wird, in der Ammoniak an einem Seitenabzug oder über Kopf abgetrennt wird,
   (2) der Sumpfaustrag aus K1 einer zweite Destillationskolonne K2 zugeführt wird, in der Wasser an einem Seitenabzug oder über Kopf abgetrennt wird,
   (3) der Sumpfaustrag aus K2 einer dritten Destillationskolonne K3 zugeführt wird, in der 1,2-PDA an einem Seitenabzug oder über Kopf abgetrennt wird,
   (4) der Sumpfaustrag aus K3 einer vierten Destillationskolonne K4 zugeführt wird, in der DMDETA im Sumpf, MIPOA an einem Seitenabzug und Leichtsieder über Kopf abgetrennt werden.

2. Verfahren nach dem vorhergehenden Anspruch, **dadurch gekennzeichnet, dass** der in Schritt (1) erhaltene Ammoniak und/oder das in Schritt (4) erhaltene MIPOA in die Umsetzung zurückgeführt werden.

3. Verfahren nach einem der vorhergehenden Ansprüche, **dadurch gekennzeichnet, dass** die katalytisch aktive Masse des Katalysators vor dessen Reduktion mit Wasserstoff sauerstoffhaltige Verbindungen des Aluminiums und sauerstoffhaltige Verbindungen des Kupfers, Nickels und/oder Kobalts enthält.

4. Verfahren nach einem der vorhergehenden Ansprüche, **dadurch gekennzeichnet, dass** die katalytisch aktive Masse des Katalysators vor dessen Reduktion mit Wasserstoff sauerstoffhaltige Verbindungen des Aluminiums, Kupfers, Nickels und Kobalts und im Bereich von 0,2 bis 5,0 Gew.-% sauerstoffhaltige Verbindungen des Zinns, berechnet als SnO, enthält.

5. Verfahren nach einem der vorhergehenden Ansprüche, **dadurch gekennzeichnet, dass** die katalytisch aktive Masse des Katalysators vor dessen Reduktion mit Wasserstoff im Bereich von 0,4 bis 4,0 Gew.-% sauerstoffhaltige Verbindungen des Zinns, berechnet als SnO, enthält.

6. Verfahren nach einem der vorhergehenden Ansprüche, **dadurch gekennzeichnet, dass** die katalytisch aktive Masse des Katalysators vor dessen Reduktion mit Wasserstoff im Bereich von 5,0 bis 35 Gew.-% sauerstoffhaltige Verbindungen des Kobalts, berechnet als CoO, enthält.

7. Verfahren nach einem der Ansprüche 1 bis 5, **dadurch gekennzeichnet, dass** die katalytisch aktive Masse des Katalysators vor dessen Reduktion mit Wasserstoff im Bereich von 10 bis 30 Gew.-% sauerstoffhaltige Verbindungen des Kobalts, berechnet als CoO, enthält.

8. Verfahren nach einem der vorhergehenden Ansprüche, **dadurch gekennzeichnet, dass** die katalytisch aktive Masse des Katalysators vor dessen Reduktion mit Wasserstoff im Bereich von

 15 bis 80 Gew.-% sauerstoffhaltige Verbindungen des Aluminiums, berechnet als $Al_2O_3$,
 1,0 bis 20 Gew.-% sauerstoffhaltige Verbindungen des Kupfers, berechnet als CuO, und
 5,0 bis 35 Gew.-% sauerstoffhaltige Verbindungen des Nickels, berechnet als NiO, enthält.

9. Verfahren nach einem der Ansprüche 1 bis 7, **dadurch gekennzeichnet, dass** die katalytisch aktive Masse des Katalysators vor dessen Reduktion mit Wasserstoff im Bereich von

 30 bis 70 Gew.-% sauerstoffhaltige Verbindungen des Aluminiums, berechnet als $Al_2O_3$,
 2,0 bis 18 Gew.-% sauerstoffhaltige Verbindungen des Kupfers, berechnet als CuO, und
 10 bis 30 Gew.-% sauerstoffhaltige Verbindungen des Nickels, berechnet als NiO, enthält.

10. Verfahren nach einem der vorhergehenden Ansprüche, **dadurch gekennzeichnet, dass** die katalytisch aktive Masse des Katalysators kein Rhenium und/oder Ruthenium enthält.

11. Verfahren nach einem der vorhergehenden Ansprüche, **dadurch gekennzeichnet, dass** die katalytisch aktive Masse des Katalysators kein Eisen und/oder Zink enthält.

12. Verfahren nach einem der vorhergehenden Ansprüche, **dadurch gekennzeichnet, dass** die katalytisch aktive Masse des Katalysators keine sauerstoffhaltigen Verbindungen des Siliziums und/oder des Zirkoniums enthält.

13. Verfahren nach einem der vorhergehenden Ansprüche, **dadurch gekennzeichnet, dass** der Absolutdruck im Bereich von 70 bis 145 bar liegt.

14. Verfahren nach einem der vorhergehenden Ansprüche, **dadurch gekennzeichnet, dass** die Reaktionstemperatur bei der Umsetzung 160-195 ° C beträgt.

15. Verfahren nach einem der vorhergehenden Ansprüche, **dadurch gekennzeichnet, dass** die Umsetzung in Gegenwart von 1,2 bis 4,5 Gew.-% Wasserstoff, bezogen auf die Menge an eingesetztem MIPOA, erfolgt.

16. Verfahren nach einem der vorhergehenden Ansprüche, **dadurch gekennzeichnet, dass** der Ammoniak in der 5 bis 30-fachen molaren Menge bezogen auf MIPOA eingesetzt wird.

## Claims

1. A process for the continuous preparation of 1,2-propylenediamine (1,2-PDA) and dimethyldiethylenetriamine (DMDETA) via reaction of monoisopropanolamine (MIPOA) with ammonia in the presence of hydrogen and a supported heterogeneous hydrogenation catalyst (catalyst), wherein the reaction is effected in a reactor in the liquid phase at an absolute pressure in the range from 60 to 170 bar and the reactor output is separated by distillation, wherein

 (1) the reactor output is supplied to a first distillation column K1 in which ammonia is removed at a side draw or overhead,
 (2) the bottoms output from K1 is supplied to a second distillation column K2 in which water is removed at a side draw or overhead,
 (3) the bottoms output from K2 is supplied to a third distillation column K3 in which 1,2-PDA is removed at a side draw or overhead,
 (4) the bottoms output from K3 is supplied to a fourth distillation column K4 in which DMDETA is removed in the bottoms, MIPOA is removed at a side draw and low boilers are removed overhead.

2. The process according to the preceding claim, wherein the ammonia obtained in step (1) and/or the MIPOA obtained in step (4) are recycled into the reaction.

**3.** The process according to either of the preceding claims, wherein the catalytically active composition of the catalyst, prior to the reduction thereof with hydrogen, comprises oxygen-containing compounds of aluminum and oxygen-containing compounds of copper, of nickel and/or of cobalt.

**4.** The process according to any of the preceding claims, wherein the catalytically active composition of the catalyst, prior to the reduction thereof with hydrogen, comprises oxygen-containing compounds of aluminum, of copper, of nickel and of cobalt and in the range from 0.2% to 5.0% by weight of oxygen-containing compounds of tin, calculated as SnO.

**5.** The process according to any of the preceding claims, wherein the catalytically active composition of the catalyst, prior to the reduction thereof with hydrogen, comprises in the range from 0.4% to 4.0% by weight of oxygen-containing compounds of tin, calculated as SnO.

**6.** The process according to any of the preceding claims, wherein the catalytically active composition of the catalyst, prior to the reduction thereof with hydrogen, comprises in the range from 5.0% to 35% by weight of oxygen-containing compounds of cobalt, calculated as CoO.

**7.** The process according to any of claims 1 to 5, wherein the catalytically active composition of the catalyst, prior to the reduction thereof with hydrogen, comprises in the range from 10% to 30% by weight of oxygen-containing compounds of cobalt, calculated as CoO.

**8.** The process according to any of the preceding claims, wherein the catalytically active composition of the catalyst, prior to the reduction thereof with hydrogen, comprises in the range from

15% to 80% by weight of oxygen-containing compounds of aluminum, calculated as $Al_2O_3$,
1.0% to 20% by weight of oxygen-containing compounds of copper, calculated as CuO, and
5.0% to 35% by weight of oxygen-containing compounds of nickel, calculated as NiO.

**9.** The process according to any of claims 1 to 7, wherein the catalytically active composition of the catalyst, prior to the reduction thereof with hydrogen, comprises in the range from

30% to 70% by weight of oxygen-containing compounds of aluminum, calculated as $Al_2O_3$,
2.0% to 18% by weight of oxygen-containing compounds of copper, calculated as CuO, and
10% to 30% by weight of oxygen-containing compounds of nickel, calculated as NiO.

**10.** The process according to any of the preceding claims, wherein the catalytically active composition of the catalyst does not comprise any rhenium and/or ruthenium.

**11.** The process according to any of the preceding claims, wherein the catalytically active composition of the catalyst does not comprise any iron and/or zinc.

**12.** The process according to any of the preceding claims, wherein the catalytically active composition of the catalyst does not comprise any oxygen-containing compounds of silicon and/or of zirconium.

**13.** The process according to any of the preceding claims, wherein the absolute pressure is in the range from 70 to 145 bar.

**14.** The process according to any of the preceding claims, wherein the reaction temperature during the reaction is 160-195°C.

**15.** The process according to any of the preceding claims, wherein the reaction is effected in the presence of 1.2% to 4.5% by weight of hydrogen, based on the amount of MIPOA used.

**16.** The process according to any of the preceding claims, wherein the ammonia is used in a 5-fold to 30-fold molar amount based on MIPOA.

## Revendications

1. Procédé pour la préparation continue de 1,2-propylènediamine (1,2-PDA) et de diméthyldiéthylènetriamine (DMDETA) par transformation de mono-isopropanolamine (MIPOA) avec de l'ammoniac en présence d'hydrogène et d'un catalyseur d'hydrogénation hétérogène supporté (catalyseur), **caractérisé en ce que** la transformation a lieu dans la phase liquide à une pression absolue dans la plage de 60 à 170 bars dans un réacteur et le produit soutiré du réacteur est séparé par distillation,

   (1) le produit soutiré du réacteur étant introduit dans une première colonne de distillation K1, dans laquelle de l'ammoniac est séparé via un soutirage latéral ou via la tête,
   (2) le produit soutiré du fond de K1 étant introduit dans une deuxième colonne de distillation K2, dans laquelle de l'eau est séparée via un soutirage latéral ou via la tête,
   (3) le produit soutiré du fond de K2 étant introduit dans une troisième colonne de distillation K3, dans laquelle de la 1,2-PDA est séparée via un soutirage latéral ou via la tête,
   (4) le produit soutiré du fond de K3 étant introduit dans une quatrième colonne de distillation K4, dans laquelle de la DMDETA est séparée dans le fond, de la MIPOA est séparée via un soutirage latéral et une fraction légère est séparée via la tête.

2. Procédé selon la revendication précédente, **caractérisé en ce que** l'ammoniac obtenu dans l'étape (1) et/ou la MIPOA obtenue dans l'étape (4) sont recyclés dans la transformation.

3. Procédé selon l'une quelconque des revendications précédentes, **caractérisé en ce que** la masse catalytiquement active du catalyseur contient, avant sa réduction avec de l'hydrogène, des composés oxygénés de l'aluminium et des composés oxygénés du cuivre, du nickel et/ou du cobalt.

4. Procédé selon l'une quelconque des revendications précédentes, **caractérisé en ce que** la masse catalytiquement active du catalyseur contient, avant sa réduction avec de l'hydrogène, des composés oxygénés de l'aluminium, du cuivre, du nickel et/ou du cobalt et dans la plage de 0,2 à 5,0% en poids de composés oxygénés de l'étain, calculé sous forme de SnO.

5. Procédé selon l'une quelconque des revendications précédentes, **caractérisé en ce que** la masse catalytiquement active du catalyseur contient, avant sa réduction avec de l'hydrogène, dans la plage de 0,4 à 4,0% en poids de composés oxygénés de l'étain, calculé sous forme de SnO.

6. Procédé selon l'une quelconque des revendications précédentes, **caractérisé en ce que** la masse catalytiquement active du catalyseur contient, avant sa réduction avec de l'hydrogène, dans la plage de 5,0 à 35% en poids de composés oxygénés du cobalt, calculé sous forme de CoO.

7. Procédé selon l'une quelconque des revendications 1 à 5, **caractérisé en ce que** la masse catalytiquement active du catalyseur contient, avant sa réduction avec de l'hydrogène, dans la plage de 10 à 30% en poids de composés oxygénés du cobalt, calculé sous forme de CoO.

8. Procédé selon l'une quelconque des revendications précédentes, **caractérisé en ce que** la masse catalytiquement active du catalyseur contient, avant sa réduction avec l'hydrogène, dans la plage de

   - 15 à 80% en poids de composés oxygénés de l'aluminium, calculé sous forme d'$Al_2O_3$,
   - 1,0 à 20% en poids de composés oxygénés du cuivre, calculé sous forme de CuO et
   - 5,0 à 35% en poids de composés oxygénés du nickel, calculé sous forme de NiO.

9. Procédé selon l'une quelconque des revendications 1 à 7, **caractérisé en ce que** la masse catalytiquement active du catalyseur contient, avant sa réduction avec l'hydrogène, dans la plage de

   - 30 à 70% en poids de composés oxygénés de l'aluminium, calculé sous forme d'$Al_2O_3$,
   - 2,0 à 18% en poids de composés oxygénés du cuivre, calculé sous forme de CuO et
   - 10 à 30% en poids de composés oxygénés du nickel, calculé sous forme de NiO.

10. Procédé selon l'une quelconque des revendications précédentes, **caractérisé en ce que** la masse catalytiquement active du catalyseur ne contient pas de rhénium et/ou de ruthénium.

**11.** Procédé selon l'une quelconque des revendications précédentes, **caractérisé en ce que** la masse catalytiquement active du catalyseur ne contient pas de fer et/ou de zinc.

**12.** Procédé selon l'une quelconque des revendications précédentes, **caractérisé en ce que** la masse catalytiquement active du catalyseur ne contient pas de composés oxygénés du silicium et/ou du zirconium.

**13.** Procédé selon l'une quelconque des revendications précédentes, **caractérisé en ce que** la pression absolue se situe dans la plage de 70 à 145 bars.

**14.** Procédé selon l'une quelconque des revendications précédentes, **caractérisé en ce que** la température de réaction lors de la transformation est de 160-195°C.

**15.** Procédé selon l'une quelconque des revendications précédentes, **caractérisé en ce que** la transformation a lieu en présence de 1,2 à 4,5% en poids d'hydrogène, par rapport à la quantité de MIPOA utilisée.

**16.** Procédé selon l'une quelconque des revendications précédentes, **caractérisé en ce que** l'ammoniac est utilisé en une quantité représentant 5 à 30 fois la quantité molaire par rapport à la MIPOA.

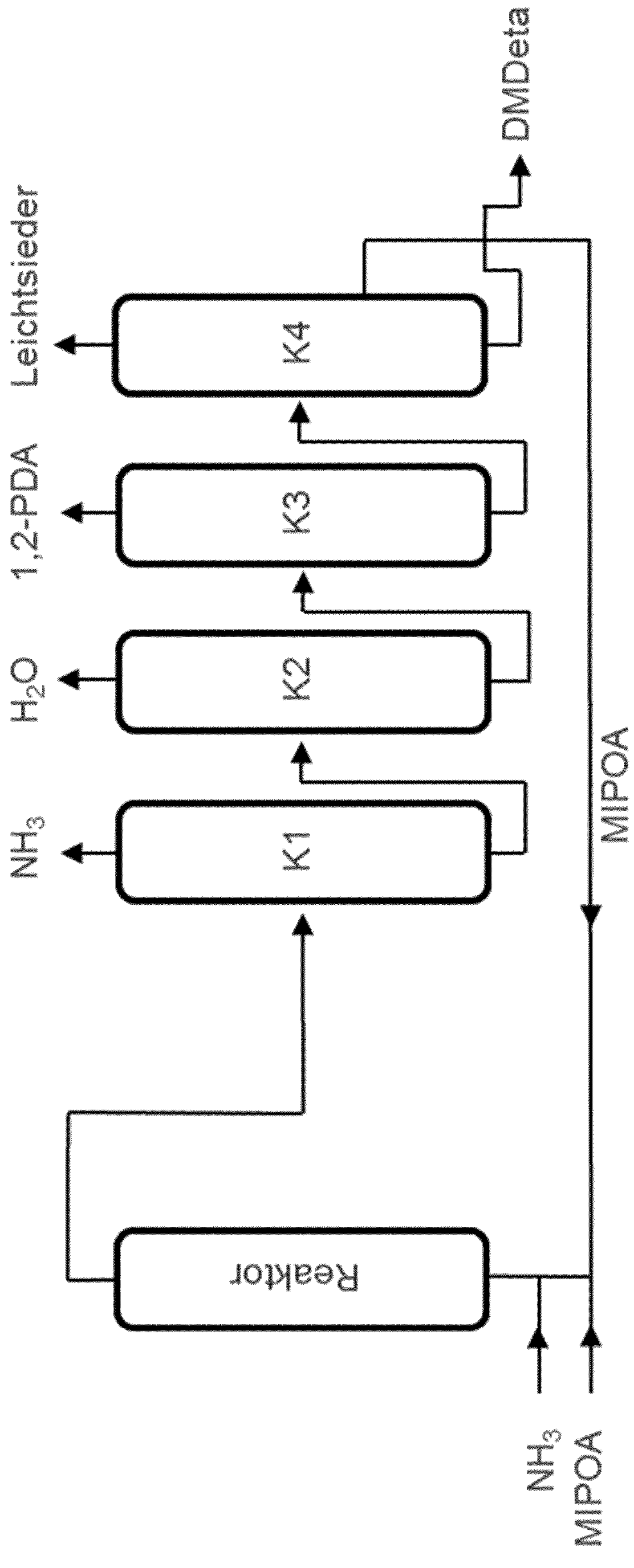
Leichtsieder
DMDeta
1,2-PDA
$H_2O$
$NH_3$
K1
K2
K3
K4
MIPOA
Reaktor
$NH_3$
MIPOA

Abbildung 1

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 03051508 A1
- WO 2008006750 A1
- WO 2009080507 A1
- WO 2009080506 A1
- WO 2009080508 A1
- WO 2009114438 A2
- WO 2011067199 A1
- CN 104693037 A
- US 2011151615 A1
- WO 2013072289 A1


### In der Beschreibung aufgeführte Nicht-Patentliteratur


- **K. V. CHERNITSKII ; V. A. BOBYLEV.** *J. Gen. Chem. USSR,* 1990, vol. 60, 1636-1643
- Fixed-Bed Reactors. Ullmann's Encyclopedia of Industrial Chemistry. vol. B 4, 199-238
- **A. B. STILES.** Catalyst Manufacture - Laboratory and Commercial Preparations. Marcel Dekker, 1983